# EUROPEAN PATENT APPLICATION

(11) **EP 4 042 940 A1**
(43) Date of publication of application: **17.08.2022**
(21) Application number: 19951116.3
(22) Date of filing: 11.11.2019
(51) Int. Cl.: A61B 5/11, A61B 5/00, A61B 5/22, A63G 31/00, A61B 5/0205

(54) **PLAY EXPERIENCE SYSTEM**

(30) Priority: 31.10.2019 KR 20190138175
(71) Applicant: Korea Electronics Technology Institute, Bundang-gu, Seongnam-si Gyeonggi-do 13509 (KR)
(72) Inventor: MOON, Yeonkug, Seoul 04743 (KR); KIM, Minjoon, Seongnam-si, Gyeonggi-do 13646 (KR); LEE, Donghyun, Seoul 08070 (KR); CHAE, Sunghun, Seoul 07663 (KR)
(74) Representative: Isarpatent
(86) International application number: PCT/KR2019/015269
(87) International publication number: WO 2021/085712

(57) **Abstract**

A play experience system disclosed in the present invention comprises an experience device including a plurality of experience rooms configured to provide spaces in which a user can carry out a play-type experience and composed of variable modules that are connected to form a course, and a plurality of experience instruments provided in each of the plurality of experience rooms for the user to experience the play-type experience so that physical development items of the user can be measured comprehensively; a sensor device comprising at least one or more sensors being disposed in a predetermined area of each of the plurality of experience instruments and configured to measure comprehensively the physical development items while the user experiences the plurality of experience instruments; and a processing device configured to receive body composition information and heart rate information of the user and comprehensive measurement information on the physical development items of the user from the at least one or more sensors, and to obtain result information on a current level of the physical development items of the user by comparing the received comprehensive measurement information on the physical development items with pre-stored standard information on the physical development items.

## Description

### [Technical Field]

The disclosure relates to a play experience system.

More specifically, the disclosure relates to a play experience system that can precisely measure physical development items of users including children and juveniles by using play-type experience devices.

### [Background Art]

In general, a person can stay healthy by being physically active along with mental activity. In the past, almost everyone moved their bodies in their everyday lives, but in recent times many people need to exercise separately because of an increase in their indoor activities.

Therefore, in places nationwide that have good scenery, trails or walking areas have been established to provide conditions for various exercises to be undertaken in a pleasant environment.

However, because people living in a city need to make many preparations in order to do exercise on trails or walking areas, and the conditions are often unfavorable, it is not easy for people living in a city to use trails or walking areas.

In addition, users that have less time for exercise mostly use gyms. If it is not feasible to use a gym, they often prepare fitness equipment at home for exercise. For this, various kinds of fitness equipment with excellent performance are available.

However, in the case of exercising alone in a gym or at home, a problem is that exercise is not undertaken continually because the user loses interest and easily becomes tired of the exercise.

Especially in the case of children, because amusement facilities for children such as play areas and jungle gyms are merely physical amusement facilities only for simple play and having fun and are composed of only facilities and equipment with a lack of professionalism and limited usage, there is also a problem in that the frequency of return visits decreases. Also, children's understanding of or accessibility to high-tech culture is degraded.

As a scheme for solving the above problems in the related art, domestic patent application publication no. 10-2019-0098870 discloses a Medi-Sportainment physical strength measurement and analysis system for play-type child growth and development, which measures physical strength, including such as body, balance, and sensation while a user has fun.

However, according to the related art document, because only one basic physical strength measurement is taken of physical strength items evaluated through physical strength measurement equipment per evaluation items provided in an experience room, there is a problem in that it is not possible to obtain information on results showing children's precise basic physical strength.

In addition, in the case that all children do not use the physical strength items evaluated by the physical strength measurement equipment per evaluation items provided in the space of the experience room, accurate measurement of the basic physical strength items is not possible; thus, there is a problem in that it is not possible to use result information showing the basic physical strength items measured as above as standard information for basic physical strength items.

Accordingly, there is a need for a new scheme for solving the above problems in the related art.

### [Disclosure]

### [Technical Problem]

The disclosure is proposed to solve the above problems in the related art, and an aspect of the disclosure is to provide a play experience system in which a plurality of experience rooms constituting an experience device for play-type experiences are configured as a plurality of variable modules disposed in the form of courses. Also, by variably configuring weight values for a user's physical development items to be measured in the plurality of experience rooms, a user's physical development items can be measured comprehensively and precisely, and standard information on the physical development items can be updated more accurately and precisely through a continuous feedback of information (data) values of the measured physical development items into existing information (data) values.

### [Technical Solution]

In order to solve the above problems, a play experience system according to an embodiment of the disclosure may include an experience device including a plurality of experience rooms configured to provide spaces in which a user can carry out a play-type experience and composed of variable modules that are connected to form a course, and a plurality of experience instruments provided in each of the plurality of experience rooms for the user to experience the play-type experience so that physical development items of the user are measured comprehensively; a sensor device including at least one or more sensors being disposed in a predetermined area of each of the plurality of experience instruments and configured to measure comprehensively the physical development items while the user experiences the plurality of experience instruments; and a processing device configured to receive body composition information and heart rate information of the user and comprehensive measurement information on the physical development items of the user from the at least one or more sensors, and to obtain result information on a current level of the physical development items of the user by comparing the received comprehensive measurement information on the physical development items with pre-stored standard information on the physical development items.

### [Advantageous Effects]

The play experience system according to the embodiment of the disclosure can achieve the following effects.
1. Because the experience device for the play-type experience is configured as variable modules disposed in the form of courses, the physical development items of the user can be measured comprehensively and precisely.
2. Because the result information on the user's physical development items measured using the plurality of experience instruments is continually updated, the result information can be used as physical development standard information.
3. Because the plurality of experience rooms are configured as variable modules, the level of difficulty of the plurality of experience instruments being provided respectively in the plurality of experience rooms can be adjusted; thus, a user having repeated experiences cannot lose interest or stop having fun.

### [Description of Drawings]

FIG. 1 is a configuration diagram schematically illustrating a play experience system according to an embodiment of the disclosure.
FIG. 2 is a diagram explaining the structure of a first experience instrument according to an embodiment of the disclosure.
FIG. 3 illustrates a state in which a star is awarded for physical development items measured comprehensively through the first experience instrument of FIG. 2.
FIG. 4 is a diagram explaining the structure of a second experience instrument according to an embodiment of the disclosure.
FIG. 5 illustrates a state in which a star is awarded for physical development items measured comprehensively through the second experience instrument of FIG. 4.
FIG. 6 is a diagram explaining the structure of a third experience instrument according to an embodiment of the disclosure.
FIG. 7 illustrates a state in which a star is awarded for physical development items measured comprehensively through the third experience instrument of FIG. 6.

### [Mode for Disclosure]

Hereinafter, preferred embodiments of the disclosure will be described in detail with reference to the accompanying drawings. In the accompanying drawings, it is to be noted that the same drawing reference numerals are used for the same elements. Further, a detailed explanation of related well-known functions or configurations incorporated herein will be omitted in the case where it is determined that they obscure the subject matter of the disclosure.

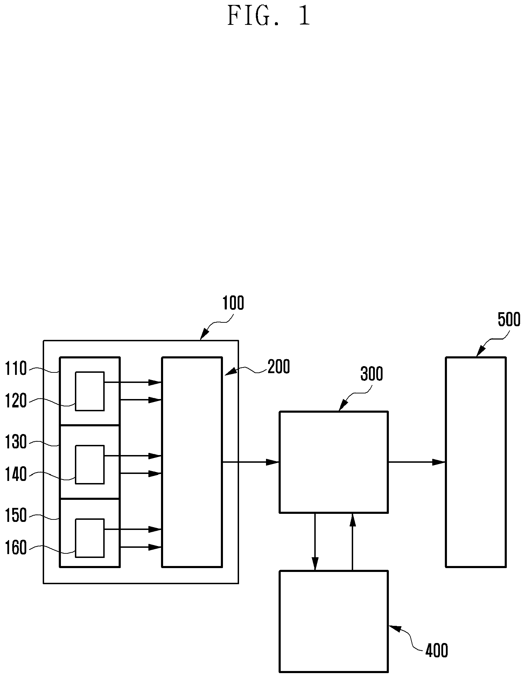

Hereinafter, a play experience system according to an embodiment of the disclosure will be described with reference to the accompanying drawings of FIGS. 1 to 7.

FIG. 1 is a configuration diagram illustrating schematically a play experience system according to an embodiment of the disclosure.

With reference to FIG. 1, a play experience system according to an embodiment of the disclosure includes a plurality of experience devices 100, a sensor device 200, and a processing device 300.

The experience device 100 according to an embodiment of the disclosure may include a plurality of experience rooms 110, 130, and 150 and a plurality of experience instruments 120, 140, and 160 provided in the plurality of experience rooms 110, 130, and 150, respectively.

The plurality of experience rooms 110, 130, and 150 may provide spaces in which a user can carry out a play-type experience, and they may be composed of variable modules which are connected to form a course.

More specifically, in configuring the play experience system, the plurality of experience rooms 110, 130, and 150 may be formed as a variable module structure so as to be disposed in various forms in accordance with the size of the space and the environmental conditions.

Each of the plurality of experience instruments 120, 140, and 160 according to an embodiment of the disclosure may be provided in the space of each of the plurality of experience rooms 110, 130, and 150 so that the user can have a play-type experience for comprehensive measurement of physical development items. Further, although the plurality of experience instruments 120, 140, and 160 may be connected in the form of courses, it is to be noted that the disclosure is not limited thereto.

In this case, each of the plurality of experience instruments 120, 140, and 160 may be implemented so that the user can undertake activities to cause the user to be interested and to have fun, and they may enable the user to measure comprehensively feature values of physical development items including muscular strength, muscular endurance, agility, a quick reaction, balance, and cardiorespiratory endurance of the user.

Weight values may be given to some or all of the physical development items being measured in the plurality of experience instruments 120, 140, and 160, and the disposition of the plurality of experience instruments 120, 140, and 160 in the experience device may be provided variably in accordance with the weight values. Based on the physical development measurement items of the user measured by each of the plurality of experience instruments 120, 140, and 160, result information on the physical development items at the current level of the user may be obtained.

Hereinafter, several examples of the plurality of experience instruments 120, 140, and 160 will be described in detail.

Meanwhile, although not illustrated, before the user enters into the first experience room 110 among the plurality of experience rooms 110, 130, and 150, the processing device 300 to be described later is provided with and stores basic personal information of the user, and the processing device 300 issues a personal membership number associated with the basic personal information. Thereafter, the user wears a wearable device (e.g., wrist band) with a recorded membership number to provide the user's bio-information, and a helmet on which a tag for safety protection and the user's location recognition is mounted. Here, the wearable device of which the membership number has been recorded may be provided in a body with the helmet.

In this case, the basic information may include a user's name, age, gender, and guardian information, and the bio-information may include information on the user's heart rate, temperature, and blood pressure. The basic information and the bio-information may be stored and managed in the processing device 300 to be described later using network communication. Further, the user's body information, such as height and weight, may be measured through body information measurement equipment disposed on the side of a pre-experience instrument to be described later, and then such information may be stored in the processing device 300.

The sensor device 200 according to an embodiment of the disclosure may be disposed in any one area or location of each of the plurality of experience instruments 120, 140, and 160 provided in each of the plurality of experience rooms 110, 130, and 150, and it may include at least one or more sensors (not illustrated) transmitting the physical development measurement information measured while the user experiences the plurality of experience instruments 120, 140, and 160 to the processing device 300 to be described later.

As for the sensor device 200, at least one of a pressure sensor, a short-range sensor, a touch sensor, and a motion detection sensor for measuring the physical development with respect to the muscular strength, muscular endurance, agility, a quick reaction, flexibility, balance, and cardiorespiratory endurance of the user is installed in each of the experience rooms and on each of the experience instruments, and it provides comprehensive measurement of the physical development items of the user while the user experiences the experience instruments 120, 140, and 160 while moving through the respective experience rooms 110, 130, and 150.

The processing device 300 according to an embodiment of the disclosure may receive the pre-measured body composition information and the heart rate information of the user and the measurement information on the physical development items of the user from the at least one or more sensors, and it may obtain the result information on the current level of the physical development items of the user by comparing the received measurement information on the physical development items with the standard information on the pre-stored physical development items. The processing device 300 may be implemented by, for example, a server, a PC, and a microcontroller, but it is to be noted that the disclosure is not limited thereto.

Meanwhile, the processing device 300 may derive the correlation between the result information and the physical development items by using the information measured by each of the plurality of experience instruments 120, 140, and 160, and the plurality of experience instruments 120, 140, and 160 provide the weight values be given to some or all of the expected physical development items through the measured information.

The processing device 300 may calculate the result information on the current level of the physical development items of the user in contrast with the standard information on the pre-stored physical development items based on the measurement information on the physical development items of the user measured through the at least one or more sensors provided in each of the plurality of experience instruments 120, 140, and 160 during the time that the user enters the first experience room 110 and exits the first experience room 110 and the pre-measured and stored heart rate change information.

Meanwhile, the standard information on the physical development items pre-stored in the processing device 300 may be obtained using a generally well-known physical activity promotion system (PAPS), and this will be explained simply as follows.

The physical development standard information for the body composition of the user may be obtained using an in-body test. In this case, using the in-body test, the user's weight, body mass index, body fat mass, and body fat percentage may be obtained. Further, the physical development standard information on the user's muscular strength may be obtained using a grip force meter; and, for example, it may be obtained by measuring the maximum value of the grip force meter for a dominant one of both hands of the user. Further, the physical development standard information on the user's muscular endurance may be obtained by performing upper body roll up; and, for example, it may be obtained by measuring the number of successes until the point of failure by an upper body roll up being performed by one cycle every 3 seconds. Further, the physical development standard information on cardiorespiratory endurance of the user may be obtained by performing a 15 m long round trip or by measuring the heart rate through a step test. Further, the physical development standard information on the user's flexibility may be obtained through forward bending of the upper body in a seated state. Further, the physical development standard information on the quick reaction of the user may be obtained through a standing long jump. Further, the physical development standard information on the agility of the user may be obtained through a total physical response test or a side-step test. Finally, the physical development standard information on the balance of the user may be obtained through the user standing on one leg with eyes closed.

Meanwhile, although not illustrated, separately from the experience device, the standard information on the physical development items, of which the correlation with some or all of the physical development items is verified, may be pre-obtained and used by using information measured through each of a plurality of pre-experience instruments (not illustrated) being provided so as to measure individually the physical development items before the user has an experience through the experience device.

The pre-experience instrument may be provided separately from the experience device 100, and it may be implemented as a structure similar to the experience device 100.

That is, the measurement information on the physical development items measured using the pre-experience instrument may be collected with respect to a plurality of users (e.g., 100 users having the same age), and information obtained by calculating an average value may be used as the standard information on the physical development items.

The standard information on the physical development items measured and obtained in the above method is stored in the processing device 300, and the processing device 300 collects the measurement information on the physical development items obtained by the user through the actual experience instruments 120, 140, and 160 from at least one or more sensors and obtains the result information on the physical development items at the current level of the user by comparing the collected measurement information on the physical development items with the pre-stored standard information on the physical development items.

Meanwhile, in the case that a user that has carried out the physical development items measures the physical development items again through the experience device 100 composed of the same experience instruments 120, 140, and 160, the processing device 300 according to an embodiment of the disclosure may adjust the experience difficulty for the experience device 100 based on the pre-stored physical development result information of the user. As an example of the difficulty adjustment of the experience device 100, the difficulty of the experience device 100 may be adjusted not only by controlling adjustment of the passage time of each of the plurality of experience rooms 110, 130, and 150 or adjustment of the time or frequency of button touch of the touch sensor, but also it may be adjusted by changing game (play) content being provided in the plurality of experience rooms 110, 130, and 150.

That is, the experience device 100 according to an embodiment of the disclosure may be implemented to be able to adjust the difficulty, and the user may not lose interest or stop having fun even if the user performs an activity repeatedly with the same experience device 100. Further, degradation of the measurement reliability, which may occur by the predicted behavior through the user's repeated experience with respect to the same configuration of the experience device 100, may be prevented.

As described above, the processing device 300 of the play experience system according to an embodiment of the disclosure may receive and store the result information on the physical development items of the user.

The processing device 300 may receive a reservation for a user and basic information on the user, and it may transmit the result information on the physical development items of the user to, for example, a personal smart device 400 or a PC (not illustrated) of the user or a guardian of the user. Accordingly, the user or the guardian of the user can easily identify and manage the result information on the physical development items of the user through the personal smart device 400 or the PC.

Meanwhile, the processing device 300 may update the result information on the physical development items obtained from a plurality of users, and it may provide the updated result information on the physical development items as the standard information on the physical development items. That is, because the result information on the physical development items obtained by the plurality of users through the experience device 100 is updated by the processing device 300, and the updated result information on the physical development items is utilized as the standard information on the physical development items, more precise result information on the physical development items of the user who experiences the experience device 100 can be obtained.

With reference to FIG. 1 again, the play experience system according to an embodiment of the disclosure may further include an immersive media device 500 disposed in each of the plurality of experience rooms 110, 130, and 150 and configured to increase visual, auditory, and tactile sensation for the user who experiences the plurality of experience instruments 120, 140, and 160.

Although FIG. 1 shows that the immersive media device 500 is provided on the outside of each of the plurality of experience rooms 110, 130, and 150, the immersive media device 500 may actually be provided on the inside of each of the plurality of experience rooms 110, 130, and 150. Further, the immersive media device 500 may be provided directly in one or more of the plurality of experience instruments 120, 140, and 160.

In the immersive media device 500, a beam projector, spotlight lighting, and LED lighting may be used as examples to increase the visual sensation for the user; a microphone and a speaker may be used as examples to increase the auditory sensation; and a mist device and a vibration device may be used as examples to increase the tactile sensation. The immersive media device 500 may be provided in the same or different combinations with respect to each of the plurality of experience rooms 110, 130, and 150 or each of the plurality of experience devices 120, 140, and 160.

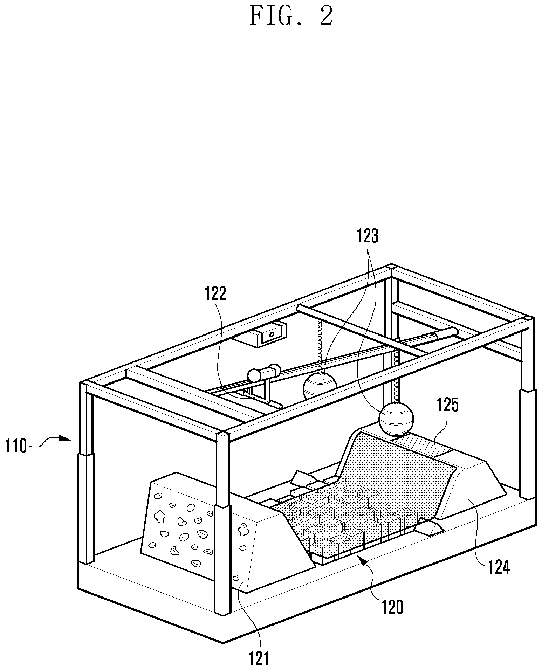

Hereinafter, with reference to FIGS. 2 to 7, several examples of the experience instruments provided in the experience rooms according to an embodiment of the disclosure will be described.

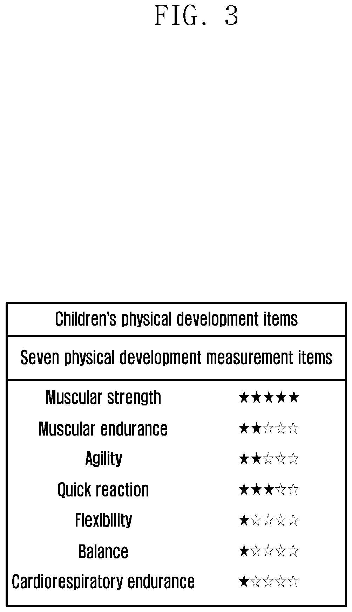

FIG. 2 shows an example of a first experience instrument in which a weight value is given to muscular strength, from among the physical development items; and FIG. 3 illustrates a state in which a star is awarded for physical development items measured using the first experience instrument of FIG. 2. Here, for convenience of explanation, the plurality of experience rooms 110, 130, and 150 may be called the first to third experience rooms 110, 130, and 150, and the plurality of experience devices 120, 140, and 160 may be called the first to third experience devices 120, 140, and 160.

With reference to FIGS. 2 and 3, the first experience instrument 120 provided in the first experience room 110 is an experience instrument implemented by giving a weight value to muscular strength, from among the physical development items; and it may measure comprehensively the physical development items. Also, it may measure specifically the muscular strength.

The first experience instrument 120 may be configured to include a zip line 122 provided in the space of the first experience room 110, a climbing apparatus 121 provided for climbing to a start location of the zip line 122, and an obstacle 123 provided on a movement line of the zip line 122. Although the illustration shows that two obstacles 123 are provided, it is to be noted that only one obstacle may be provided.

The first experience instrument 120 enables the user to climb up to the start location of the zip line 122 by using a plurality of holders provided on the surface of the climbing apparatus 121, to touch the obstacles 123 with his or her feet during a descent movement along with gripping handles of the zip line 122 from the start location of the zip line 122, and then to land accurately on an arrival location so that the physical development items of the user can be measured comprehensively. Success can be recorded and the success record stored in the processing device 300 only in the case that the user has performed successfully all of the above-described processes (climbing process, descent movement process, obstacle touching process, and landing process) after entering into the first experience instrument 120, whereas failure is recorded and the failure record is not stored in the processing device 300 in the case that even one process is not performed successfully.

Although not illustrated, at the entrance and at the exit of the first experience room 110, a motion detection sensor configured to sense the entry/exit of a user and a timer configured to check the entry/exit time may be installed, and on the plurality of holders of the climbing apparatus and on the handles of the zip line 122, a pressure sensor configured to sense the grip of the user may be installed. On the obstacles 123, impact sensors configured to sense whether two feet of the user have accurately touched the obstacles 123 (e.g., whether an impact over a predetermined level has been transferred to the obstacles 123) may be installed.

Further, in the first experience room 110, an immersive media device may be installed. Specifically, as the immersive media device, an LED spotlight for notifying entry/success/failure and a speaker for outputting sound may be installed, and a beam projector for outputting a background video for realization while the zip line 122 moves may be installed. Further, a mist spray device spraying mist when the user has arrived at the arrival point may be installed. In this case, success/failure-related background music may be output through the speaker.

A play experience process using the first experience instrument 120 in the first experience room 110 will be described. Whether the user enters into or exits from the first experience room 110 is sensed through the motion detection sensor, and the time from entry to exit is measured by the timer.

After the entry, the user climbs to the start location of the zip line 122 by gripping the plurality of holders of the climbing apparatus 121. The user moves up to the arrival point of the zip line 122 by gripping the handles of the zip line 122. In this case, the gripping of the holder of the climbing apparatus 121 and the handles of the zip line 122 by the user is sensed by the pressure sensor.

Meanwhile, the impact sensor senses whether the user has applied an impact over the predetermined level to the obstacles 123 using his or her two feet while moving along the zip line 122. Thereafter, if the user has landed in a landing area 125 of a landing zone 124, whether the user has landed accurately is sensed by the pressure sensor (not illustrated) installed on the landing area 125.

If the experience of the first experience instrument 120 is completed through the above-described processes, the agility and the quick reaction of the user, from among the physical development items, may be measured during the time recorded from entry to exit of the first experience instrument 120; the muscular endurance may be measured from the activity of climbing the climbing apparatus 121 and moving along the zip line 122; and the muscular strength may be measured from the activity of the user kicking the obstacles 123 with his or her feet.

As illustrated in FIG. 3, in the experience through the comprehensive activity of the first experience instrument 120, a high star rating is given to the muscular strength having the highest weight value, from among the physical development items. Further, because the remaining physical development items (e.g., the flexibility, balance, and cardiorespiratory endurance of the user) excluding the main measurement items are measured comprehensively by the comprehensive activity through the first experience instrument 120, a star may be awarded for the remaining physical development items.

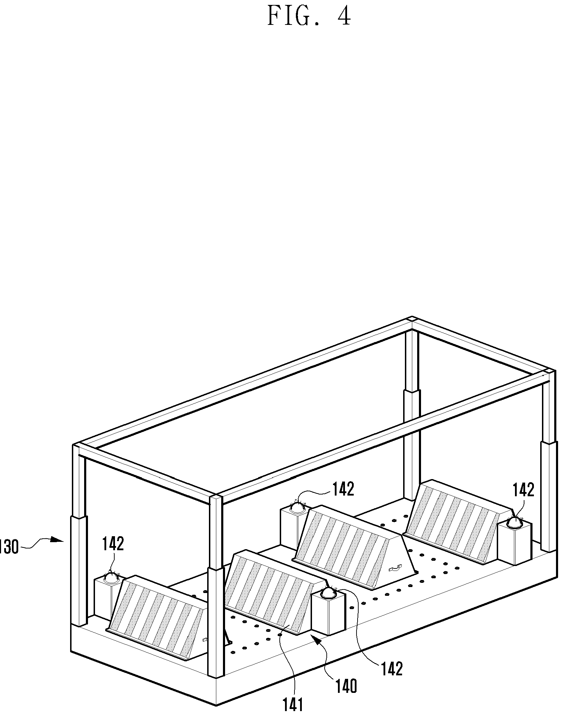

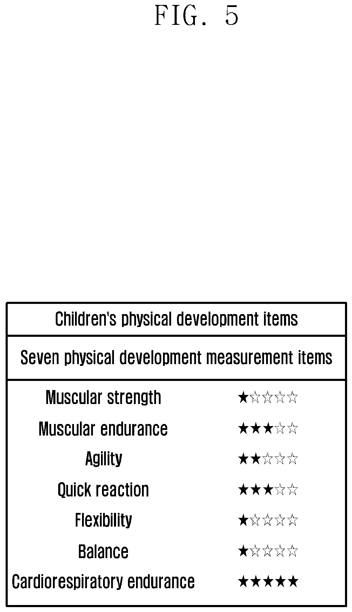

FIG. 4 shows an example of a second experience instrument in which a weight value is given to the cardiorespiratory endurance of the user, from among the physical development items, and FIG. 5 illustrates a state in which a star is awarded for physical development items measured through the second experience instrument of FIG. 4.

With reference to FIGS. 4 and 5, the second experience instrument 140 provided in the second experience room 130 is an experience instrument implemented by giving a weight value to the cardiorespiratory endurance of the user, from among the physical development items; and it may measure comprehensively the physical development items. Also, it may measure intensively the cardiorespiratory endurance of the user.

Although not illustrated, it is preferable that the entrance of the second experience room 130 is disposed to be connected to the exit of the first experience room 110 so that the user having experienced the first experience instrument 120 can experience successively the second experience instrument 140.

The second experience instrument 140 as above may include a plurality of zigzag obstacles 141 provided in the space of the second experience room 130, and on one side of each of the plurality of zigzag obstacles 141, a touch button 142 may be provided.

The second experience instrument 140 enables the user to touch the touch button 142 installed on one side of each of the plurality of zigzag obstacles 141 while moving in a zigzag through the plurality of zigzag obstacles 141; thus, the physical development items of the user can be measured comprehensively. If the second experience instrument 140 is completed within a predetermined time, success can be recorded and the success record stored in the processing device 300, whereas failure is recorded and the failure record is not stored in the processing device 300 if the time expires before the user touches all touch buttons 142, if the touch buttons 142 are not touched sequentially, or if the user passes over the zigzag obstacles 141.

Although not illustrated, at the entrance and at the exit of the second experience room 130, a motion detection sensor configured to sense the entry/exit of a user and a timer configured to check the entry/exit time may be installed. In this case, a touch sensor may be installed on each of the touch buttons 142.

Further, in the second experience room 130, an immersive media device may be installed. As the immersive media device, an LED spotlight for notifying entry/success/failure and a speaker for outputting sound may be installed, and an LED for an effect to notify of the remaining time may be installed. In this case, success/failure-related background music may be output through the speaker.

A play experience process using the second experience instrument 140 in the second experience room 130 will be described. Whether the user enters into or exits from the second experience room 130 is sensed through the motion detection sensor, and the time from entry to exit is measured by the timer.

After the entry into the second experience room 130, if the user touches the touch button 142 installed on one side of each of the plurality of zigzag obstacles 141 while moving in the space between the plurality of zigzag obstacles 141, the touch sensor senses the touch of the user.

As described above, if the experience of the second experience instrument 140 is completed, the agility and the quick reaction of the user, from among the physical development items, are measured during the time recorded from entry to exit of the second experience instrument 140, and the muscular endurance is measured from the activity of passing through the plurality of zigzag obstacles 141.

As illustrated in FIG. 5, in the experience through the comprehensive activity of the second experience instrument 140, a high star rating is given to the muscular endurance having the highest weight value, from among the physical development items. Further, since the remaining physical development items (e.g., the muscular strength, flexibility, and balance) excluding the main measurement items are measured comprehensively by the comprehensive activity through the second experience instrument 140, a star may be awarded for the remaining physical development items.

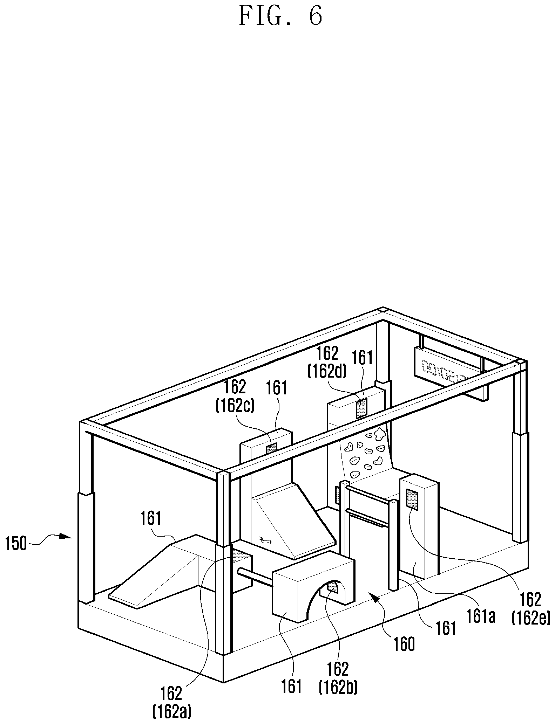

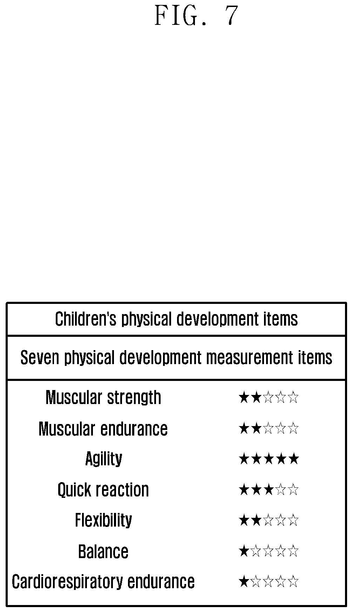

FIG. 6 shows an example of a third experience instrument 160 in which a weight value is given to the agility, from among the physical development items, and FIG. 7 illustrates a state in which a star is awarded for physical development items measured through the third experience instrument of FIG. 6.

With reference to FIGS. 6 and 7, the third experience instrument 160 provided in the third experience room 150 is an experience instrument implemented by giving a weight value to agility, from among the physical development items; and it may measure comprehensively the physical development items. Also, it may measure specifically the agility.

Although not illustrated, it is preferable that the entrance of the third experience room 150 is disposed to be connected to the exit of the second experience room 130 so that the user having experienced the first experience instrument 120 or the second experience instrument 140 can experience successively the third experience instrument 160.

The third experience instrument 160 as above may include a plurality of fixtures 161 provided in various shapes in the space of the third experience room 150. The plurality of fixtures 161 may include palm pads 162 (162a to 162d) directly provided on blocks constituting the respective fixtures 161, or a palm pad 162 (162a) provided on a separate block 161a. For example, the plurality of fixtures 161 may include a fixture 161 for the user to touch the palm pad 161a on the block, a fixture 161 for the user to touch the palm pad 162b while lying on the floor or bending over, a fixture 161 for the user to touch the palm pad 162c while climbing to the top of a slope, a fixture 161 for the user to touch the palm pad 162d while climbing, and a fixture 162 for the user to touch the palm pad 162 provided on a separate block with another hand or foot while hanging on a bar with one hand or both hands, but the disclosure is not limited thereto. Further, the respective palm pads 162 may be implemented in a lighting method.

The third experience instrument 160 enables the user to measure comprehensively the physical development items of the user by entering the third experience room 150 and by touching the palm pad 162 directly provided to the plurality of fixtures 161 or separately provided. Success may be recorded and the success record stored in the processing device 300 only in the case that the user touches all the palm pads 162 within a predetermined time, whereas failure is recorded and the failure record is not stored in the processing device 300 in the case that the user is unable to touch even one of the palm pads 162.

Although not illustrated, at the entrance and at the exit of the third experience room 150, a motion detection sensor configured to sense the entry/exit of a user and a timer configured to check the entry/exit time may be installed, and a touch sensor may be installed on each of the palm pads 162.

Further, in the third experience room 150, an immersive media device 500 may be installed. As the immersive media device, an LED spotlight for notifying entry/success/failure and a speaker for outputting sound may be installed, an LED billboard for counting the time to touch the palm pad 162 may be installed, and a lighting device capable of adjusting brightness in the third experience room 150 may be installed. In this case, success/failure-related background music may be output through the speaker.

A play experience process using the third experience instrument 160 in the third experience room 150 will be described. Whether the user enters into or exits from the third experience room 150 is sensed through the motion detection sensor, and the time from entry to exit is measured by the timer.

After the entry of the user, the user touches the palm pads 162 provided on the plurality of fixtures 161. If the user touches the palm pad 162, the touch sensor senses the touch.

If the experience of the third experience instrument 160 is completed through the above-described process, the agility and the quick reaction of the user, from among the physical development items, are measured during the time recorded from entry to exit of the third experience instrument 160; and, in particular, the agility is measured from the activity of quickly touching the palm pad 162, and the quick reaction and the muscular endurance are measured from the activity of quickly moving between the plurality of fixtures 161.

As illustrated in FIG. 7, in the experience through the comprehensive activity of the third experience instrument 160, a high star rating is given to the agility having the highest weight value, from among the physical development items. Further, because the remaining physical development items (e.g., the balance and the cardiorespiratory endurance of the user) excluding the main measurement items are measured comprehensively by the comprehensive activity through the third experience instrument 160, a star may be awarded even for the remaining physical development items.

As described above, for example, not only the first experience instrument 120 to the third experience instrument 160 but also more experience instruments in which weight values are given to the respective physical development items may be provided in the plurality of experience rooms 110, 130, and 150 or in additional experience rooms (not illustrated).

Meanwhile, the processing device 300 can obtain the result information based on the measurement information on the physical development items measured comprehensively and obtained through the plurality of experience instruments (the first to third experience instruments 120, 140, and 160 illustrated in FIGS. 2 to 7). Accordingly, the user can obtain more precise result information on the physical development items.

### [Industrial Applicability]

Since various modified examples can be made by the configuration and the method described and exemplified in the specification without deviating from the scope of the disclosure, all matters included in the detailed description or illustrated in the accompanying drawings are exemplary, and are not intended to limit the scope of the disclosure. Accordingly, the scope of the disclosure should not be limited by the above-described exemplary embodiment, but it should be determined only by the appended claims and their equivalents.

## Claims

1. A play experience system comprising:
an experience device including a plurality of experience rooms configured to provide spaces in which a user can carry out a play-type experience and composed of variable modules that are connected to form a course, and a plurality of experience instruments provided in each of the plurality of experience rooms for the user to experience the play-type experience so that physical development items of the user are measured comprehensively;
a sensor device including at least one or more sensors being disposed in a predetermined area of each of the plurality of experience instruments and configured to measure comprehensively the physical development items while the user experiences the plurality of experience instruments; and
a processing device configured to receive body composition information and heart rate information of the user and comprehensive measurement information on the physical development items of the user from the at least one or more sensors, and to obtain result information on a current level of the physical development items of the user by comparing the received comprehensive measurement information on the physical development items with pre-stored standard information on the physical development items.

2. The play experience system of claim 1, wherein the physical development items comprise muscular strength, muscular endurance, agility, a quick reaction, flexibility, balance, and cardiorespiratory endurance of the user.

3. The play experience system of claim 2, wherein the processing device is configured to derive a correlation between the result information and the physical development items by using the information measured by each of the plurality of experience instruments,
wherein the plurality of experience instruments gives weight values to some or all of the physical development items which are expected through the information measured by each of the plurality of experience instruments, and
wherein the plurality of experience instruments are variably disposed in accordance with the weight values.

4. The play experience system of claim 3, wherein the processing device is configured to calculate the result information on the current level of the physical development items of the user in contrast with the pre-stored standard information on the physical development items, based on the measurement information on the physical development items, which are measured through the at least one or more sensors provided in each of the plurality of experience instruments during the time that the user enters a first experience room and exits a last experience room among the plurality of experience rooms, and heart rate change information.

5. The play experience system of claim 3, wherein, as the standard information on the physical development items, i) predetermined standard information obtained using a physical activity promotion system (PAPS) is used, or ii) pre-obtained standard information is used, wherein the pre-obtained standard information is verified in terms of a correlation with some or all of the physical development items, by using the information measured through each of a plurality of experience instruments, which is provided separately from the experience device and capable of measuring the physical development items before the user experiences the experience device.

6. The play experience system of claim 5, wherein the processing device is configured to update a plurality of pieces of result information on the physical development items obtained from a plurality of users, and the updated plurality of pieces of result information is used as the standard information on the physical development items.

7. The play experience system of claim 6, wherein in the case that the user that has carried out the physical development items measures the physical development items again using the same experience device, experience difficulty of the experience device is able to be adjusted based on the result information on the pre-stored physical development items of the user.

8. The play experience system of claim 1, wherein the sensor device comprises at least one of a pressure sensor, a short-range sensor, a touch sensor, and a motion detection sensor.

9. The play experience system of claim 1, further comprising an immersive media device disposed in each of the plurality of experience rooms and configured to increase at least one of visual tactility, auditory tactility, or tactile sensation for the user.
